# EUROPÄISCHE PATENTANMELDUNG

(11) **EP 0 976 411 A1**
(43) Veröffentlichungstag der Anmeldung: **02.02.2000**
(21) Anmeldenummer: 99111680.7
(22) Anmeldetag: 16.06.1999
(51) Int. Cl.: A61L 9/03

(54) **Vorrichtung zur Abgabe von Duftstoffen**

(30) Priorität: 28.07.1998 DE 29813461 U
(71) Anmelder: Voit, Hans, 82166 Gräfelfing (DE)
(72) Erfinder: Hans Voit, 82166 Gräfelfing (DE)
(74) Vertreter: Haft, von Puttkamer, Berngruber, Czybulka

(57) **Zusammenfassung**

Vorrichtung zur Abgabe von Duftstoffen, bei der in einem Gehäuse (1) ein Vorratsbehälter (17) für eine Duftstofflösung, eine mit einer elektrischen Heizeinrichtung (19) erwärmbare, nach oben offene Verdampfungsschale (18), ein über der Verdampfungsschale angeordneter Ventilator (21), der die Duftstoffe durch Luftaustrittsöffnungen (9) im Gehäuse (1) nach außen bläßt, und eine Dosierpumpe (23) vorgesehen sind, mit der die Duftstofflösung von dem Vorratsbehälter (17) der Verdampfungsschale (18) zugeführt wird, wobei das Gehäuse (1) zur Befestigung an einer Wand oder Decke ausgebildet ist.

## Beschreibung

Die Erfindung bezieht sich auf eine Vorrichtung zur Abgabe von Duftstoffen nach dem Oberbegriff des Anspruchs 1.

Eine derartige Vorrichtung ist bekannt (deutsches Gebrauchsmuster 93 08 368). Die bekannte Vorrichtung weist ein etwa hüfthohes säulenförmiges Gehäuse auf, das am Boden abgestellt wird. Die Luftaustrittsöffnungen, durch die die Duftstoffe austreten, sind am oberen Ende der Säule vorgesehen.

Die bekannte Vorrichtung hat sich zwar im Großen und Ganzen bewährt. Die Duftverteilung in einem Raum läßt jedoch noch zu wünschen übrig.

Aufgabe der Erfindung ist es, eine derartige Duftstoffabgabevorrichtung bereitzustellen, die zu einer deutlich verbesserten Duftstoffverteilung führt.

Dies wird erfindungsgemäß dadurch erreicht, dass das Gehäuse zur Befestigung an einer Wand oder Decke ausgebildet ist.

Die Vorrichtung kann damit über Kopfhöhe angeordnet werden. Da die Duftstoffe schwerer als Luft sind, sinken sie nach unten, wobei sie sich verteilen. Damit läßt sich in Kopfhöhe eine optimale Duftstoffkonzentration erhalten. Eine besonders bevorzugte Stelle zur Befestigung der erfindungsgemäßen Vorrichtung ist oberhalb einer Tür oder dergleichen Zutrittsstelle. Dadurch wird der Duft beim Eintritt in den betreffenden Raum besonders deutlich wahrgenommen.

Nachstehend ist eine bevorzugte Ausführungsform der erfindungsgemäßen Vorrichtung anhand der Zeichnung beispielhaft näher erläutert. Darin zeigen:
- Fig. 1: eine Vorderansicht der Vorrichtung mit abgenommener Vorderwand; und
- Fig. 2: eine Seitenansicht der Vorrichtung.

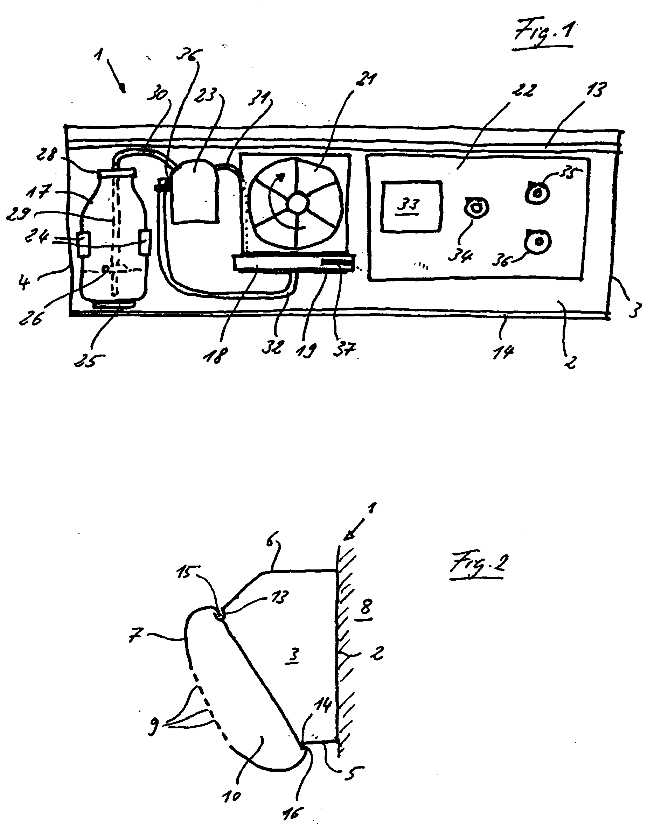

Die Vorrichtung weist ein waagrecht angeordnetes, langgestrecktes, kastenförmiges Gehäuse 1 mit einer Rückwand 2, Seitenwänden 3, 4, einer Bodenwand 5, einer oberen Abdeckwand 6 und einer Vorderwand 7 auf. Mit der Rückwand 2 ist das Gehäuse 1 an einer Wand 8 des betreffenden Gehäuses befestigt. Die Vorderwand 7 ist als nach außen gewölbte Wanne mit Seitenwänden 10 ausgebildet, die zusammen mit den Seitenwänden 3, 4 das Gehäuse 1 seitlich verschließen. Die Vorderwand 7 ist zumindest im mittleren Bereich mit Luftaustrittsöffnungen 9 versehen, wie in Fig. 2 angedeutet. Das Gehäuse 1, einschließlich der Vorderwand 7, kann beispielsweise aus Blech bestehen oder aus Kunststoff.

Damit die Vorrichtung die Duftstoffe schräg nach unten von der Wand 8 zur Mitte des betreffenden Raumes abgibt, ist die Vorderwand 7 schräg nach unten gerichtet. Dazu sind die Seitenwände 3, 4 abgeschrägt, und die obere Abdeckwand 6 ist im vorderen Bereich nach unten abgewinkelt.

Die Vorderwand 7 ist abnehmbar ausgebildet. Dazu ist an der vorderen Längskante der oberen Abdeckwand 6 und der Bodenwand 5 jeweils ein Winkelprofil 13, 14 vorgesehen, in dem die Vorderwand 7 mit ihren Längskanten 15, 16 befestigbar ist.

In dem einen, linken Endbereich des Gehäuses 1 ist ein Vorratsbehälter 17 für eine Duftstofflösung angeordnet, und im mittleren Bereich eine nach oben offene Verdampfungsschale 18, die durch eine an ihrer Unterseite vorgesehene Widerstandsheizung 19 z.B. in Form einer Heizspirale beheizbar ist. Oberhalb der Verdampfungsschale 18 ist ein Ventilator 21 angeordnet, durch den die in der beheizten Verdampfungsschale 18 verdampften Duftstoffe durch die Luftaustrittsöffnungen 9 in der Vorderwand 7 nach außen geblasen werden.

Der Schaltkasten 22 zur Steuerung der Vorrichtung ist an dem anderen, rechten Endbereich des länglichen Gehäuses 1 angeordnet. Zwischen dem Vorratsbehälter 17 und der Verdampfungsschale 18 mit dem Ventilator 21 ist eine Dosierpumpe 23 angeordnet.

Der Vorratsbehälter 17, die Dosierpumpe 23, die Verdampfungsschale 18 und der Schaltkasten 22 sind an der Rückwand 2 des Gehäuses 1 befestigt, und der Ventilator 21 am vorderen Bereich der Verdampfungsschale 18.

Die Vorratsflasche 17 wird durch zwei Federbügel 24 gehalten und steht mit ihrem Boden auf einem Halter 25. Der Behälter 17 ist mit einer induktiven Füllstandsmeßvorrichtung versehen. Dazu weist der Halter 25 eine nicht dargestellte elektrische Spule auf, während in dem Behälter 17 ein in Fig. 1 gestrichelt dargestellter Schwimmer 26 mit einem Magneten vorgesehen ist.

Durch den Deckel 28 des Behälters 17 ragt ein Saugrohr 29 nach unten. Das obere Ende des Saugrohres 29 ist mit einem Schlauch 30 mit der Dosierpumpe 23 verbunden, um die Duftstofflösung anzusaugen und dosiert über den Schlauch 31 an die Schale 18 abzugeben und zwar, wie in Fig. 1 gestrichelt dargestellt, an deren Rand.

Die zylindrische Schale 18 weist einen (nicht dargestellten) zu ihrer Mitte hin leicht abfallenden Boden und in der Mitte des Bodens eine Austrittsöffnung auf. An diese Austrittsöffnung ist ein Schlauch 32 angeschlossen, der mit seinem freien Ende oberhalb der Schale 18 an der Dosierpumpe 23 mit einem Klemmhalter 36 befestigt ist.

Der Schaltkasten 22 ist mit einer Anzeige 33 zur Einstellung der Betriebszeit der Vorrichtung (beispielsweise den Geschäftszeiten), mit einem Ein/Aus-Schalter 34, einem Drehknopf 35 zur Steuerung der Dosierpumpe 23 und einem Drehknopf 36 zur Steuerung des Ventilators 21 versehen.

Durch Einstellung der entsprechenden Geschwindigkeit der Dosierpumpe 23 wird die Duftstofflösung dem Rand der Verdampfungsschale 18 zugeführt, und zwar mit einer solchen Geschwindigkeit, dass sie möglichst vollständig verdampft, bevor sie die Austrittsöffnung in der Mitte des Bodens erreicht. Um eine möglichst konstante Verdampfungsgeschwindigkeit zu gewährleisten, wird die Temperatur der Heizung 29 einem Thermostaten 37 geregelt, der, wie in Fig. 1 angedeutet, in einer Bohrung angeordnet ist, die sich vom Umfang der z.B. aus einer massiven Aluminiumscheibe gebildeten Verdampfungsschale 18 nach innen erstreckt.

Falls die induktive Füllstandsmeßvorrichtung (Schwimmer 26) das Unterschreiten eines vorgegebenen Mindestfüllstands anzeigt, wird die Dosierpumpe 23 abgeschaltet. Falls die Duftstofflösung in der Schale 18 nicht vollständig verdampft, wird sie von dem Schlauch 32 aufgenommen, der zugleich als Aufnahmebehälter dient. In erster Linie dient der Schlauch 32 jedoch dazu, beim Reinigen der Verdampfungsschale 18 die Reinigungsflüssigkeit abfließen zu lassen. Eine Reinigung ist beispielsweise erforderlich, wenn die Vorrichtung auf einen anderen Duft umgestellt werden soll.

Die vorstehend beschriebene Vorrichtung stellt nur ein Ausführungsbeispiel dar. So kann das Gehäuse beispielsweise auch als Rohr ausgebildet sein, das an beiden Seiten verschlossen ist. Bei einem solchen Rohr kann dann der Vorratsbehälter liegend angeordnet und seitlich einführbar sein. Er kann damit wesentlich größer ausgeführt werden. Ferner kann die Verdampfungsschale als langgestrecktes Rechteck ausgebildet und der darüber angeordnete Ventilator ein sich entlang der rechteckigen Verdampfungsschale erstreckender Walzenlüfter sein. Dadurch kann die abgegebene Duftstoffmenge wesentlich erhöht werden.

## Patentansprüche

1. Vorrichtung zur Abgabe von Duftstoffen, bei der in einem Gehäuse ein Vorratsbehälter für eine Duftstofflösung, eine mit einer elektrischen Heizeinrichtung erwärmbare, nach oben offene Verdampfungsschale, ein über der Verdampfungsschale angeordneter Ventilator, der die Duftstoffe durch Luftaustrittsöffnungen im Gehäuse nach außen bläst, und eine Dosierpumpe vorgesehen sind, mit der die Duftstofflösung von dem Vorratsbehälter der Verdampfungsschale zugeführt wird, dadurch gekennzeichnet, dass das Gehäuse (1) zur Befestigung an einer Wand (8) oder Decke ausgebildet ist.

2. Vorrichtung nach Anspruch 1 oder 2, dadurch gekennzeichnet, dass das Gehäuse (1) länglich ausgebildet und waagrecht an der Wand (8) oder Decke befestigbar ist.

3. Vorrichtung nach Anspruch 1 oder 2, dadurch gekennzeichnet, dass das Gehäuse (1) eine Rückwand (2) aufweist, mit der es an der Wand (8) oder Decke befestigbar ist.

4. Vorrichtung nach Anspruch 2, dadurch gekennzeichnet, dass der Vorratsbehälter (17) an einem Endbereich des Gehäuses (1) und die Verdampfungsschale (18) mit dem Ventilator (21) im mittleren Bereich des Gehäuses (1) angeordnet sind.

5. Vorrichtung nach Anspruch 4, dadurch gekennzeichnet, dass an dem anderen Endbereich des länglichen Gehäuses (1) ein Schaltkasten (22) angeordnet ist.

6. Vorrichtung nach einem der vorstehenden Ansprüche, dadurch gekennzeichnet, dass im Boden der Verdampfungsschale (18) eine Austrittsöffnung zum Spülen der Schale (18) vorgesehen ist.

7. Vorrichtung nach Anspruch 6, dadurch gekennzeichnet, dass an der Austrittsöffnung ein Schlauch (32) angeschlossen ist, der mit seinem freien Ende oberhalb der Schale befestigbar ist.

8. Vorrichtung nach einem der vorstehenden Ansprüche, dadurch gekennzeichnet, dass der Vorratsbehälter (17) eine Füllstandsmeßeinrichtung aufweist.

9. Vorrichtung nach Anspruch 8, dadurch gekennzeichnet, dass die Füllstandsmeßvorrichtung die Dosierpumpe (23) bei Unterschreiten eines vorgegebenen Füllstandes abschaltet.

10. Vorrichtung nach Anspruch 8 oder 9, dadurch gekennzeichnet, dass die Füllstandsmeßvorrichtung durch eine induktive Füllstandsmeßvorrichtung mit einem Magneten an einem Schwimmer (26) gebildet wird.
